# EUROPEAN PATENT APPLICATION

(11) **EP 3 831 369 A1**
(43) Date of publication of application: **09.06.2021**
(21) Application number: 19213378.3
(22) Date of filing: 03.12.2019
(51) Int. Cl.: A61K 31/01, A61K 31/136, A61K 31/137, A61K 31/225, A61K 31/277, A61K 31/397, A61K 31/4704, A61K 31/573, A61K 31/575, A61K 31/7076, A61K 38/02, A61K 38/21, A61K 39/395, A61P 25/00, A61P 25/28

(54) **SQUALENE FOR THE TREATMENT OF DEMYELINATING DISORDERS**

(71) Applicant: Max-Planck-Gesellschaft zur Förderung der Wissenschaften e.V., 80539 München (DE)
(72) Inventor: The designation of the inventor has not yet been filed
(74) Representative: Arth, Hans-Lothar

(57) **Abstract**

The present invention relates to the squalene as pharmaceutically active agent and/or pharmaceutical composition comprising the squalene for use in the prophylaxis and/or treatment of demyelinating disorders/diseases, in particular, multiple sclerosis.

## Description

### Specification

The present invention relates to the squalene as pharmaceutically active agent and/or pharmaceutical composition comprising the squalene for use in the prophylaxis and/or treatment of demyelinating disorders/diseases, in particular multiple sclerosis.

### Background of the invention

The term demyelination describes a loss of myelin with relative preservation of axons. This results from diseases that damage myelin sheaths or the cells that form them. Demyelinating disorders/diseases can be classified according to their pathogenesis into several categories: demyelination due to inflammatory processes, viral demyelination, demyelination caused by acquired metabolic derangements, hypoxic-ischemic forms of demyelination and demyelination caused by focal compression.

Demyelinating disorders/diseases is caused by/associated with demyeliation and is selected from the group consisting of: Lysosomes and Lysosomal Disorders, Metachromatic Leukodystrophy, Multiple Sulfatase Deficiency, Globoid Cell Leukodystrophy (Krabbe Disease), GM1 Gangliosidosis, GM2 Gangliosidosis, Fabry Disease, Fucosidosis, Mucopolysaccharidoses, Free Sialic Acid Storage Disorder, Neuronal Ceroid Lipofuscinoses, Adult Polyglucosan Body Disease, Peroxisomes and Peroxisomal Disorders, Peroxisome Biogenesis Defects, Peroxisomal D-Bifunctional Protein Deficiency, Peroxisomal Acyl-CoA Oxidase Deficiency, X-linked Adrenoleukodystrophy, Refsum Disease, Mitochondria and Mitochondrial Disorders, Mitochondrial Encephalopathy with Lactic Acidosis and Stroke-like Episodes, Leber Hereditary Optic Neuropathy, Kearns-Sayre Syndrome, Mitochondrial Neurogastrointestinal Encephalomyopathy, Leigh Syndrome and Mitochondrial Leukoencephalopathies, Pyruvate Carboxylase Deficiency, Multiple Carboxylase Deficiency, Cerebrotendinous Xanthomatosis, Cockayne Syndrome, Trichothiodystrophy with Photosensitivity, Pelizaeus-Merzbacher Disease and X-linked Spastic Paraplegia Type 2, 18q- Syndrome, Phenylketonuria, Glutaric Aciduria Type 1, Propionic Acidemia, Nonketotic Hyperglycinemia, Maple Syrup Urine Disease, 3-Hydroxy 3-Methylglutaryl-CoA Lyase Deficiency, Canavan Disease, L-2-Hydroxyglutaric Aciduria, D-2-Hydroxyglutaric Aciduria, Hyperhomocysteinemias, Urea Cycle Defects, Serine Synthesis Defect Caused by 3-Phosphoglycerate Dehydrogenase Deficiency, Molybdenum Cofactor Deficiency and Isolated Sulfite Oxidase Deficiency, Galactosemia, Sjogren-Larsson Syndrome, Lowe Syndrome, Wilson Disease, Menkes Disease, Fragile X Premutation, Hypomelanosis of Ito, Incontinentia Pigmenti, Alexander Disease, Giant Axonal Neuropathy, Megalencephalic Leukoencephalopathy with Subcortical Cysts, Congenital Muscular Dystrophies, Myotonic Dystrophy Type , Myotonic Dystrophy Type 2, X-linked Charcot-Marie-Tooth Disease, Oculodentodigital Dysplasia, Leukoencephalopathy with Vanishing White Matter, Aicardi-Goutières Syndrome, Leukoencephalopathy with Calcifications and Cysts, Leukoencephalopathy with Brain Stem and Spinal Cord Involvement and Elevated White Matter Lactate, Hypomyelination with Atrophy of the Basal Ganglia and Cerebellum, Hereditary Diffuse Leukoencephalopathy with Neuroaxonal Spheroids, Dentatorubropallidoluysian Atrophy, Cerebral Amyloid Angiopathy, Cerebral Autosomal Dominant Arteriopathy with Subcortical Infarcts and Leukoencephalopathy, Cerebral Autosomal Recessive Arteriopathy with Subcortical Infarcts and Leukoencephalopathy, Polycystic Lipomembranous Osteodysplasia with Sclerosing Leukoencephalopathy (Nasu-Hakola Disease), Pigmentary Orthochromatic Leukodystrophy, Adult-Onset Autosomal Dominant Leukoencephalopathies, Inflammatory and Infectious Disorders, Multiple Sclerosis, Optic neuritis, Neuromyelitis optica, Transverse myelitis, Acute Disseminated Encephalomyelitis, Acute hemorrhagic leucoencephalitisAdrenoleukodystrophy and adrenomyeloneuropathy, Progressive Multifocal Leukoencephalopathy, Central Pontine and Extrapontine Myelinolysis, Hypoxic-ischemic demyelination, Alzheimre's disease (AD), Acute Hemorrhagic Encephalomyelitis, Acquired Immunodeficiency Syndrome, Brucellosis, Subacute Sclerosing Panencephalitis, Congenital and Perinatal Cytomegalovirus Infection, Whipple Disease, Toxic Encephalopathies, Iatrogenic Toxic Encephalopathies, Hypernatremia, Marchiafava-Bignami Syndrome, Posterior Reversible Encephalopathy Syndrome, Langerhans Cell Histiocytosis, Post-Hypoxic-Ischemic Damage, Post-Hypoxic-Ischemic Leukoencephalopathy of Neonates, Neonatal Hypoglycemia, Delayed Posthypoxic Leukoencephalopathy, White Matter Lesions of the Elderly Subcortical Arteriosclerotic Encephalopathy, Vasculitis, Leukoencephalopathy and Dural Venous Fistula, Leukoencephalopathy after Chemotherapy and/or Radiotherapy, Gliomatosis Cerebri, Diffuse Axonal Injury, Wallerian Degeneration and Myelin Loss Secondary to Neuronal and Axonal Degeneration.

Preferred types of demyelinating disease and their causes include as follows:
- Multiple sclerosis (MS) - the most common demyelinating disease of the central nervous system
- Optic neuritis - inflammation of the optic nerve in one or both eyes
- Neuromyelitis optica (Devic's disease) - inflammation and demyelination of the central nervous system, especially of the optic nerve and spinal cord
- Transverse myelitis - inflammation of the spinal cord
- Acute disseminated encephalomyelitis (ADEM) - inflammation of the brain and spinal cord
- Adrenoleukodystrophy and adrenomyeloneuropathy - rare, inherited metabolic disorders
- Acute hemorrhagic leucoencephalitis (AHL) / AHL may be preceded by viral or *M pneumoniae* infection.
- Progressive multifocal leukoencephalopathy (PML) - PML is caused by the papovavirus, JC virus
- Central pontine myelinolysis (CPM) and extrapontine myelinolysis (EPM) - CPM and EPM are acquired metabolic demyelination diseases. CPM is a monophasic demyelinating disease of the pons and lower midbrain
- Hypoxic-ischemic demyelination - the myelinating oligodendrocyte bears the brunt of the hypoxic or ischemic damage
- Alzheimer's disease (AD) - Myelin loss has been observed consistently in AD.

Multiple sclerosis (MS) is an inflammatory disease of the central nervous system that is characterized by foci of inflammation and damage of myelin membranes which are the insulating covers of nerve cell fibers in the nervous system. The disease affects the ability of parts of the nervous system to communicate and results in physical, mental, and sometimes psychiatric problems. For example, MS patients often have vision disorders, such as blindness in one eye or double vision. Patients also exhibit muscle weakness, trouble with sensation, and trouble with coordination.

The underlying mechanism of MS pathology is thought to be either destruction by the immune system (outside-in hypothesis) or failure of the neural cells likely myelin-synthesizing cells (inside-out hypothesis). The cause of the disease is not known, but may include genetics and environmental factors such as viral infections. MS is usually diagnosed based on analysis of a patient's symptoms and the results of supporting medical tests.

Current treatments attempt to improve symptoms after an attack and prevent new attacks. Medications used to treat MS are only modestly effective and can have side effects and be poorly tolerated. Physical therapy can help with a patient's ability to function. Because of the lack of good alternatives, many patients pursue unproven alternative treatments. Life expectancy is 5 to 10 years lower than that of an unaffected population.

Myelin is a multi-layered extension of the plasma membrane of myelin-synthesizing cells, i.e. oligodendrocytes in the central nervous system and Schwann cells in the peripheral nervous system. Myelin membranes that contain approximately 70% of lipids and for approximately 30% of proteins, wrap around neuronal axons, which acts as an electrical isolator, which increases the speed of a nerve impulse. The destruction of the myelin sheath leads to the occurrence of demyelination and inflammatory responses.

Despite the diversity of available therapeutic agents in this area, there remains a need to develop effective approaches for the treatment of multiple sclerosis. This is necessary first of all in order to increase the arsenal of possible therapeutic agents for the choice of the attending doctor, including providing new therapeutic opportunities for patients who don't respond or don't respond satisfactorily to existing therapy, or who suffer from side effects of such therapy, and also to offer new possibilities of combination therapy, offering new therapeutic approaches that can be combined with existing ones to achieve the above goals. It is also necessary to reduce the cost of treatment for patients, since the drugs available on the market are usually expensive.

It is the objective of the present invention to provide new therapeutic compound for effective treatment of demyelinating disorders/diseases. The present invention teaches that the squalene as pharmaceutically active agent and/or pharmaceutical composition comprising the squalene is useful in the prophylaxis and/or treatment of demyelinating disorders/diseases, especially, demyelinating diseases associated with microglial activation/inflammation selected from multiple sclerosis (MS), optic neuritis, neuromyelitis optica (Devic's disease), transverse myelitis, acute disseminated encephalomyelitis (ADEM), adrenoleukodystrophy, adrenomyeloneuropathy, acute hemorrhagic leucoencephalitis (AHL), progressive multifocal leukoencephalopathy (PML), central pontine myelinolysis (CPM), extrapontine myelinolysis (EPM), hypoxic-ischemic demyelination, and Alzheimer's disease, in particular, multiple sclerosis (MS).

The objective of the present invention is solved by teaching of the independent claims. Further advantageous features, aspects and details of the invention are evident from the dependent claims, the description, the figures, and the examples of the present application.

### Description of the invention

The present invention relates to squalene for use in treatment and/or prophylaxis of demyelinating disease associated with microglial activation/inflammation selected from multiple sclerosis (MS), optic neuritis, neuromyelitis optica (Devic's disease), transverse myelitis, acute disseminated encephalomyelitis (ADEM), adrenoleukodystrophy, adrenomyeloneuropathy, acute hemorrhagic leucoencephalitis (AHL), progressive multifocal leukoencephalopathy (PML), central pontine myelinolysis (CPM), extrapontine myelinolysis (EPM), hypoxic-ischemic demyelination, and Alzheimer's disease, in particular, multiple sclerosis.

Squalene is a natural triterpene known as an important intermediate of cholesterol/phytosterol biosynthesis in animal and plant organisms. Squalene has the following chemical fomula (I) and the IUPAC name is (6E,10E,14E,18E)-2,6,10,15,19,23-hexamethyltetracosa-2,6,10,14,18,22-hexaene

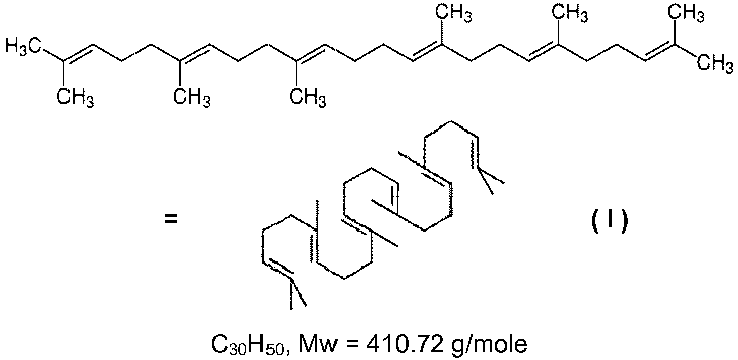

Squalene is an essential intermediate of cholesterol biosynthesis. Cholesterol biosynthesis follows the mevalonate (MVA)/isoprenoid pathway. It starts with the conversion of acetyl-CoA to 3-hydroxy-3-methylglutaryl coenzyme A (HMG-CoA), followed by the reduction to MVA with 3-hydroxy-3-methylglutaryl coenzyme A reductase (HMGR). This is the rate limiting step, highly regulated through the HMGR activation or degradation. The formation of the isoprenoids isopentenyl diphosphate (IPP) and dimethylallyl diphosphate (DMAPP) occurs after phosphorylation and decarboxylation of mevalonate, DMAPP being a precursor of all polyprenyl compounds. The subsequent condensations with additional IPPs will conduct to geranyl pyrophosphate and farnesyl pyrophosphate (FPP) which are involved in the synthesis of isoprenylated cellular metabolites. Two FPPs can further condense to squalene and subsequently lead to the synthesis of sterols, steroids, and bile acids.

The term "multiple sclerosis" or "MS," as used herein, refers to an inflammatory disease affecting the nervous system, in which the myelin sheaths around the axons of the brain and spinal cord are damaged, leading to demyelination and scarring as well as a broad spectrum of clinical signs and symptoms. MS may be classified into different disease subtypes, including relapsing/remitting MS (RRMS), secondary progressive MS, primary progressive MS, and progressive relapsing MS. The relapsing-remitting subtype may be characterized by unpredictable relapses followed by periods of months to years of relative quiet (remission) with no new signs of disease activity. The relapsing-remitting subtype may usually begin with a clinically isolated syndrome (CIS). In CIS, a patient may have an attack suggestive of demyelination. Often CIS marks the onset of MS.

A diagnosis of multiple sclerosis can be established on the basis of established clinical symptoms and the clinical symptoms are well known to the skilled person.

The clinical symptoms of multiple sclerosis may include vision problems, dizziness, vertigo, sensory dysfunction, weakness, problems with coordination, loss of balance, fatigue, pain, neurocognitive deficits, mental health deficits, bladder dysfunction, bowel dysfunction, sexual dysfunction, heat sensitivity.

The term "multiple sclerosis" also refers to any other autoimmune disease manifested by demyelination of the central nervous system's neurons. The first symptoms which appear at the onset of MS may be referred to at times as "MS-related symptoms." The symptoms of MS in EAE-induced animals (animal model of MS) may be typically weakness and malfunction in the animal's tail, followed by weakness of its rear feet and finally weakness in its front feet. In humans, such first MS-related symptoms may typically be double vision, facial numbness, facial weakness, vertigo, nausea, vomiting ataxia, weakness of the arms, and others.

Four basic MS disease courses (also called types or phenotypes) have been defined by the International Advisory Committee on Clinical Trials of MS in 2013: clinically isolated syndrome, relapsing remitting, secondary progressive and primary progressive (Neurology, 2014, pp.278-286).

Clinically Isolated Syndrome (CIS) is a first episode of neurologic symptoms caused by inflammation and demyelination in the central nervous system. The episode, which by definition must last for at least 24 hours, is characteristic of multiple sclerosis but does not yet meet the criteria for a diagnosis of MS because people who experience a CIS may or may not go on to develop MS. When CIS is accompanied by lesions on a brain MRI (magnetic resonance imaging) that are similar to those seen in MS, the person has a high likelihood of a second episode of neurologic symptoms and diagnosis of relapsing-remitting MS. When CIS is not accompanied by MS-like lesions on a brain MRI, the person has a much lower likelihood of developing MS.

Relapsing-remitting MS (RRMS) is the most common disease course and characterized by clearly defined attacks of new or increasing neurologic symptoms. These attacks - also called relapses or exacerbations - are followed by periods of partial or complete recovery (remissions). During remissions, all symptoms may disappear, or some symptoms may continue and become permanent. Despite the fact that there is no apparent progression of the disease during the periods of remission, worsening of disease expression such as histopathological changes likely occur. At different points in time, RRMS can be further characterized as either active (with relapses and/or evidence of new MRI activity) or not active, as well as worsening (a confirmed increase in disability over a specified period of time following a relapse) or not worsening. Approximately 85 percent of people with MS are initially diagnosed with RRMS.

Primary progressive MS (PPMS) is characterized by worsening neurologic function (accumulation of disability) from the onset of symptoms, without early relapses or remissions. PPMS can be further characterized at different points in time as either active (with an occasional relapse and/or evidence of new MRI activity) or not active, as well as with progression (evidence of disease worsening on an objective measure of change over time, with or without relapse or new MRI activity) or without progression. Approximately 15 percent of people with MS are diagnosed with PPMS.

Secondary progressive MS (SPMS) follows an initial relapsing-remitting course. Most people who are diagnosed with RRMS will eventually transition to a secondary progressive course in which there is a progressive worsening of neurologic function (accumulation of disability) over time. SPMS can be further characterized at different points in time as either active (with relapses and/or evidence of new MRI activity) or not active, as well as with progression (evidence of disease worsening on an objective measure of change over time, with or without relapses) or without progression.

The term "treating" or "treatment," as used herein, refers to amelioration of some of the undesired manifestations and/or disease expressions of multiple sclerosis, the prevention of the manifestation of such symptoms before they occur, slowing down or completely preventing the progression of the disease (as may be evident by longer periods between reoccurrence episodes, slowing down or prevention of the deterioration of symptoms etc.), enhancing the onset of the remission period, extending the period of new MRI brain lesions, slowing down the irreversible damage caused in the progressive-chronic stage of the disease (both in the primary and secondary stages), delaying the onset of said progressive stage, or a combination of two or more of the above.

The "effective amount," as used herein, refers to the amount of a required to ameliorate the symptoms of a disease relative to an untreated patient. The effective amount of active compound(s) used to practice the present invention for therapeutic treatment of a disease varies depending upon the manner of administration, the age, body weight, and general health of the subject. Ultimately, the attending physician or veterinarian will decide the appropriate amount and dosage regimen. Such amount is referred to as an "effective" amount.

The term "patient" or "subject," as used herein, refers to a mammalian subject (primates (e.g. , humans, cows, sheep, goats, pigs, horses, dogs, cats, rabbits, rats, mice and the like), preferably a human subject, that has, is suspected of having, or is or may be susceptible to a condition associated with multiple sclerosis. In one embodiment, the present method may be used for treating a patient who suffers from multiple sclerosis, e.g., with any symptoms as discussed above. In another embodiment, the present method may also be used to prevent a perspective patient from getting multiple sclerosis.

Therefore, the present invention refers to squalene for use in treatment and/or prophylaxis of demyelinating disorders/diseases selected from the group consisting of:
Lysosomes and Lysosomal Disorders, Metachromatic Leukodystrophy, Multiple Sulfatase Deficiency, Globoid Cell Leukodystrophy (Krabbe Disease), GM1 Gangliosidosis, GM2 Gangliosidosis, Fabry Disease, Fucosidosis, Mucopolysaccharidoses, Free Sialic Acid Storage Disorder, Neuronal Ceroid Lipofuscinoses, Adult Polyglucosan Body Disease, Peroxisomes and Peroxisomal Disorders, Peroxisome Biogenesis Defects, Peroxisomal D-Bifunctional Protein Deficiency, Peroxisomal Acyl-CoA Oxidase Deficiency, X-linked Adrenoleukodystrophy, Refsum Disease, Mitochondria and Mitochondrial Disorders, Mitochondrial Encephalopathy with Lactic Acidosis and Stroke-like Episodes, Leber Hereditary Optic Neuropathy, Kearns-Sayre Syndrome, Mitochondrial Neurogastrointestinal Encephalomyopathy, Leigh Syndrome and Mitochondrial Leukoencephalopathies, Pyruvate Carboxylase Deficiency, Multiple Carboxylase Deficiency, Cerebrotendinous Xanthomatosis, Cockayne Syndrome, Trichothiodystrophy with Photosensitivity, Pelizaeus-Merzbacher Disease and X-linked Spastic Paraplegia Type 2, 18q- Syndrome, Phenylketonuria, Glutaric Aciduria Type 1, Propionic Acidemia, Nonketotic Hyperglycinemia, Maple Syrup Urine Disease, 3-Hydroxy 3-Methylglutaryl-CoA Lyase Deficiency, Canavan Disease, L-2-Hydroxyglutaric Aciduria, D-2-Hydroxyglutaric Aciduria, Hyperhomocysteinemias, Urea Cycle Defects, Serine Synthesis Defect Caused by 3-Phosphoglycerate Dehydrogenase Deficiency, Molybdenum Cofactor Deficiency and Isolated Sulfite Oxidase Deficiency, Galactosemia, Sjogren-Larsson Syndrome, Lowe Syndrome, Wilson Disease, Menkes Disease, Fragile X Premutation, Hypomelanosis of Ito, Incontinentia Pigmenti, Alexander Disease, Giant Axonal Neuropathy, Megalencephalic Leukoencephalopathy with Subcortical Cysts, Congenital Muscular Dystrophies, Myotonic Dystrophy Type , Myotonic Dystrophy Type 2, X-linked Charcot-Marie-Tooth Disease, Oculodentodigital Dysplasia, Leukoencephalopathy with Vanishing White Matter, Aicardi-Goutières Syndrome, Leukoencephalopathy with Calcifications and Cysts, Leukoencephalopathy with Brain Stem and Spinal Cord Involvement and Elevated White Matter Lactate, Hypomyelination with Atrophy of the Basal Ganglia and Cerebellum, Hereditary Diffuse Leukoencephalopathy with Neuroaxonal Spheroids, Dentatorubropallidoluysian Atrophy, Cerebral Amyloid Angiopathy, Cerebral Autosomal Dominant Arteriopathy with Subcortical Infarcts and Leukoencephalopathy, Cerebral Autosomal Recessive Arteriopathy with Subcortical Infarcts and Leukoencephalopathy, Polycystic Lipomembranous Osteodysplasia with Sclerosing Leukoencephalopathy (Nasu-Hakola Disease), Pigmentary Orthochromatic Leukodystrophy, Adult-Onset Autosomal Dominant Leukoencephalopathies, Inflammatory and Infectious Disorders, Multiple Sclerosis, Optic neuritis, Neuromyelitis optica, Transverse myelitis, Acute Disseminated Encephalomyelitis, Acute hemorrhagic leucoencephalitisAdrenoleukodystrophy and adrenomyeloneuropathy, Progressive Multifocal Leukoencephalopathy, Central Pontine and Extrapontine Myelinolysis, Hypoxic-ischemic demyelination, Alzheimre's disease (AD), Acute Hemorrhagic Encephalomyelitis, Acquired Immunodeficiency Syndrome, Brucellosis, Subacute Sclerosing Panencephalitis, Congenital and Perinatal Cytomegalovirus Infection, Whipple Disease, Toxic Encephalopathies, Iatrogenic Toxic Encephalopathies, Hypernatremia, Marchiafava-Bignami Syndrome, Posterior Reversible Encephalopathy Syndrome, Langerhans Cell Histiocytosis, Post-Hypoxic-Ischemic Damage, Post-Hypoxic-Ischemic Leukoencephalopathy of Neonates, Neonatal Hypoglycemia, Delayed Posthypoxic Leukoencephalopathy, White Matter Lesions of the Elderly Subcortical Arteriosclerotic Encephalopathy, Vasculitis, Leukoencephalopathy and Dural Venous Fistula, Leukoencephalopathy after Chemotherapy and/or Radiotherapy, Gliomatosis Cerebri, Diffuse Axonal Injury, Wallerian Degeneration and Myelin Loss Secondary to Neuronal and Axonal Degeneration.

Preferred, the present invention refers to squalene for use in treatment and/or prophylaxis of demyelinating disease associated with microglial activation/inflammation selected from multiple sclerosis (MS), optic neuritis, neuromyelitis optica (Devic's disease), transverse myelitis, acute disseminated encephalomyelitis (ADEM), adrenoleukodystrophy, adrenomyeloneuropathy, acute hemorrhagic leucoencephalitis (AHL), progressive multifocal leukoencephalopathy (PML), central pontine myelinolysis (CPM), extrapontine myelinolysis (EPM), hypoxic-ischemic demyelination, and Alzheimer's disease.

More Preferred, the present invention refers to squalene for use in treatment and/or prophylaxis of Alzheimer's disease and multiple sclerosis (MS).

Still more preferred, the present invention refers to squalene for use in treatment and/or prophylaxis of multiple sclerosis (MS) including clinically isolated syndrome (CIS), relapsing-remitting multiple sclerosis (RRMS), primary progressive multiple sclerosis (PPMS) and secondary progressive multiple sclerosis (SPMS).

Surprisingly, it was found in the present invention that squalene alone as active pharmaceutical ingredient is useful for prophylaxis and treatment of the above-mentioned demyelinating disorders/diseases, in particular multiple sclerosis.

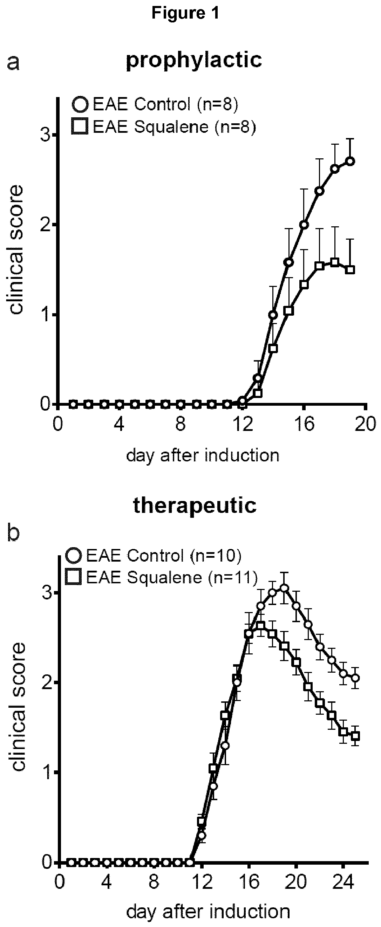

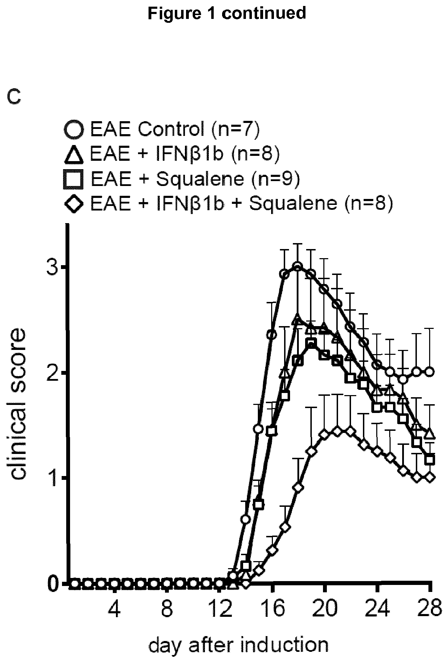

Experiment 1 shows that the squalene is useful for prophylaxis and treatment of multiple sclerosis. These results are shown in Figure 1A -1B. Most of all, it is proven that without any further active agent, the squalene is alone effectively used for prophylaxis and treatment of multiple sclerosis. Compared to interferon beta-1b, the first line standard drug for MS treatment, it was demonstrated that the treatment of multiple sclerosis alone with squalene shows more effective prophylactic effect than the treatment of multiple sclerosis with interferon beta-1b as shown in Figure 1C.

In particular, it is proven that the squalene effectively inhibits the inflammation and demyelination in the central nervous system. It is shown that squalene that is an intermediate of cholesterol biosynthesis has the potential to ameliorate disease severity in a mouse model of immune mediated myelin disease (EAE) in a prophylactic (Figure 1a) and therapeutic (Figure 1b) treatment paradigm.
In addition, squalene administration further reduced disease severity in a combined treatment with daily injections of the first line standard drug in Multiple Sclerosis, interferon beta-1b (Figure 1c). Such synergistic effect is unexpected. These data show that squalene and interferon target different disease processes.

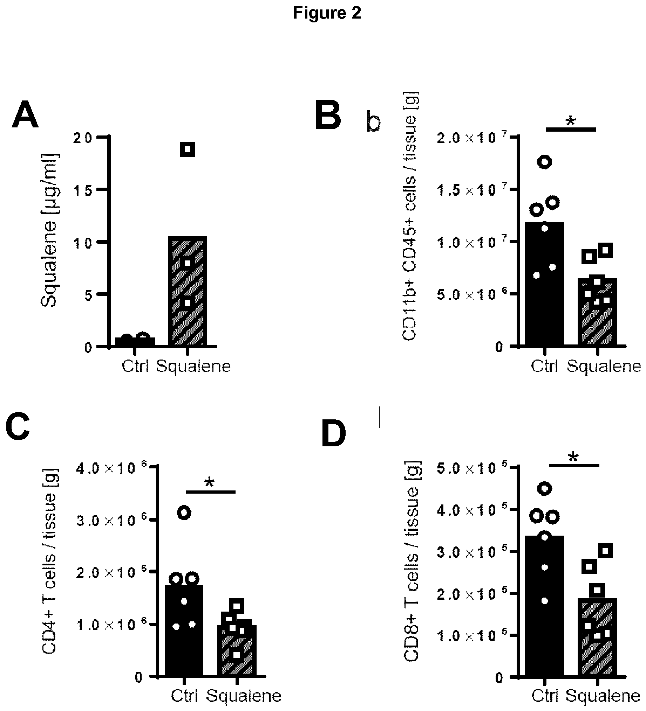

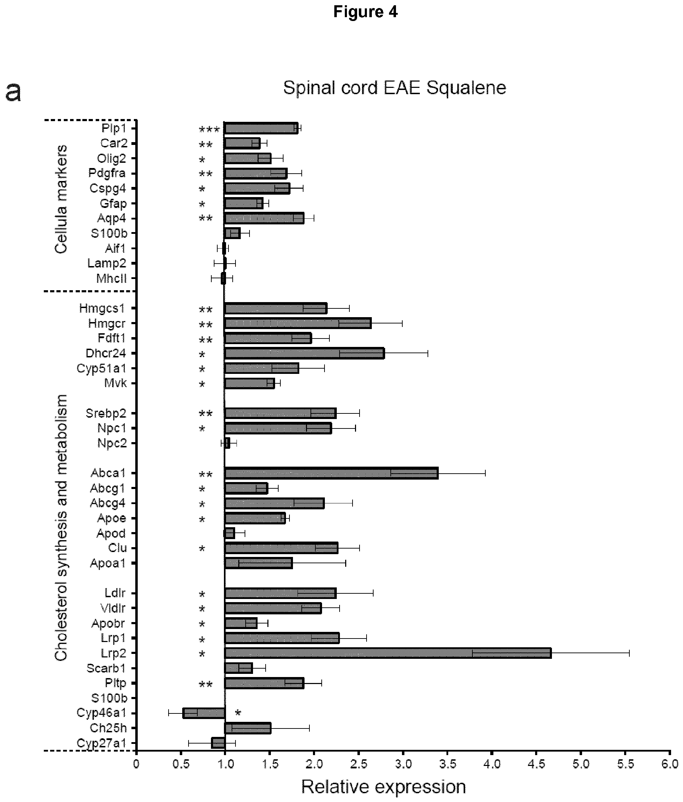

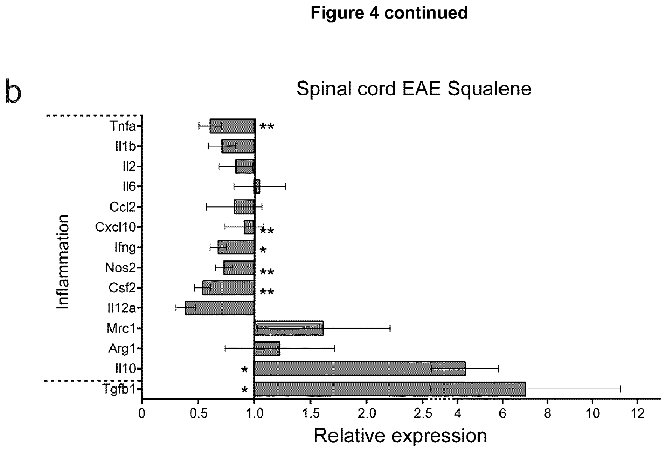

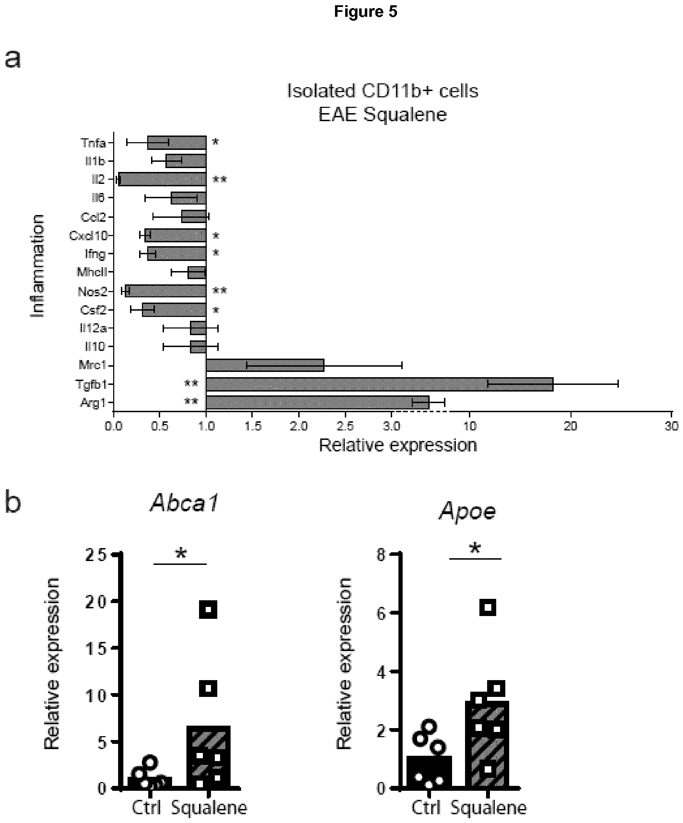

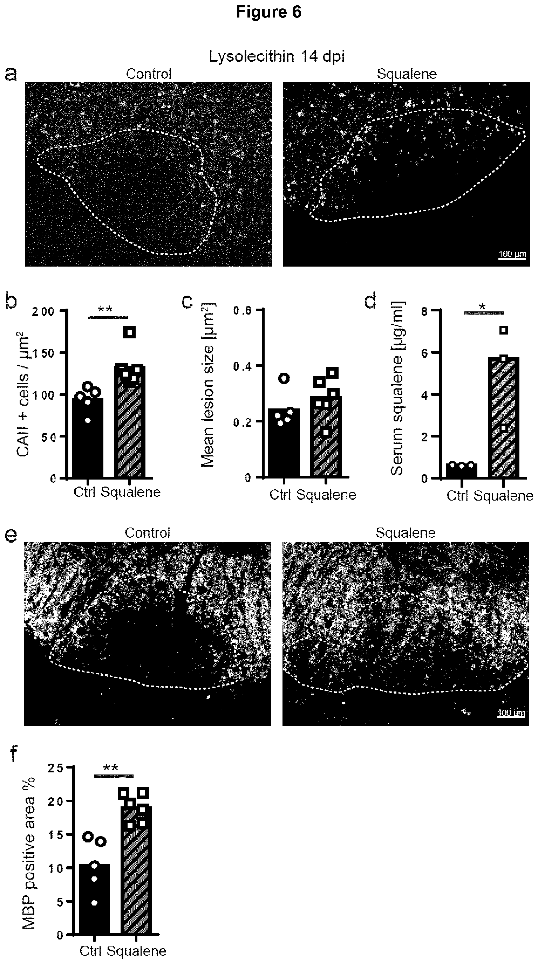

Dietary squalene administration leads to a marked increase in serum squalene in the two disease models tested (Figure 2a, 6e).
It is found that squalene administration reduced numbers of inflammatory CD4+ and CD8+ T cells as well as CD11b+/CD45+ myeloid cells at peak of disease (Figure 2 b-d). These data indicate that squalene therapy leads to a reduction of recruited inflammatory cells into the spinal cord parenchyma and likely an amelioration of the inflammatory milieu within the CNS.
By targeted expression profiling in spinal cord of EAE treated mice, we found significantly increased expression of genes related to myelination and oligodendrocyte differentiation (Plp1, Car2, Olig2, Pdgfra, Cspg4), as well as cholesterol synthesis (Hmgc1, Hmgcr, Fdft1, Dhcr24, Cyp51a1, Mvk), cholesterol export (Abca1, Abcg1, Abcg4, Apoe, Clu) and cholesterol uptake (Ldlr, Vldlr, Apobr, Lrp1, Lrp2) in squalene supplemented mice compared to chow fed controls (Figure 4a).
In addition, we detected reduced expression of pro-inflammatory mediator genes while gene expression of the anti-inflammatory molecules 1110 and Tgfb1 was significantly increased (Figure 4b).
It is also demonstrated that dietary squalene supplementation has a direct impact on tissue microglia/macrophages during EAE, inducing a shift to an anti-inflammatory phenotype and increased cholesterol efflux that supports tissue repair (Figures 5a and 5b).

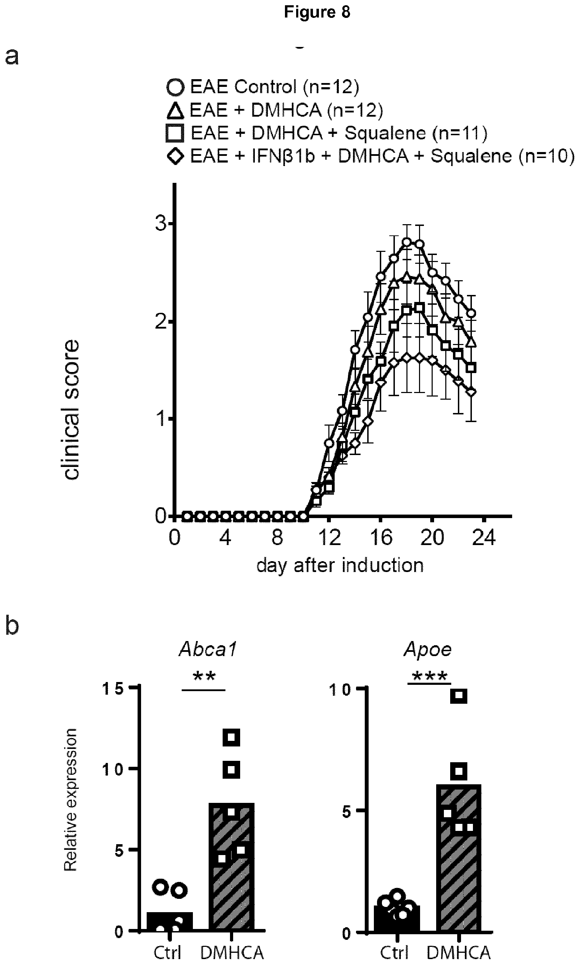

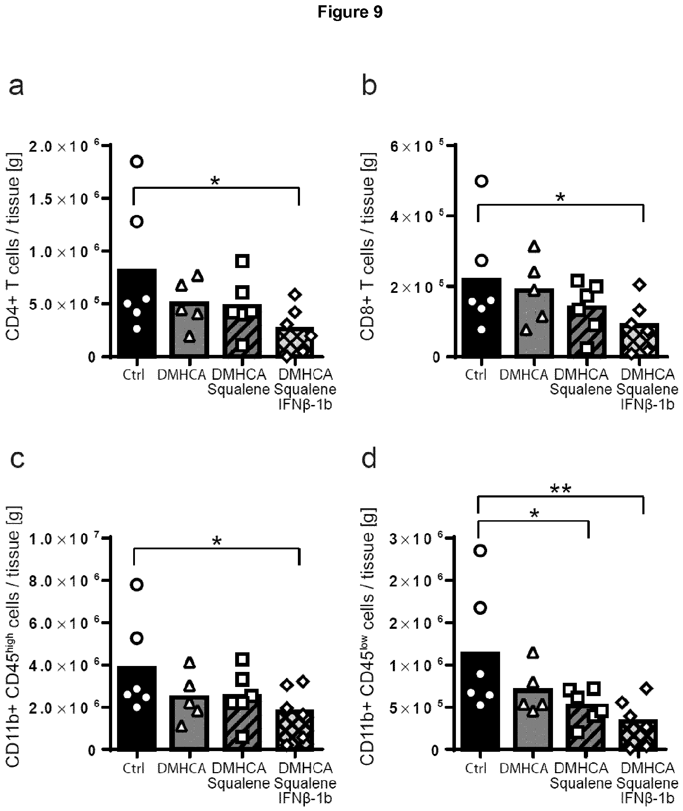

Furthermore, it is found that administration of squalene in combination with a LXR agonist, in particular, N,N-dimethyl-3β-hydroxycholenamide (DMHCA) shows a synergistic effect and thus potentiates the efficiency of anti-inflammatory therapy due to its pro-remyelinating effect on oligodendrocytes and inflammation dampening effect on microglia/macrophages (Figures 8a, 9a-d).
Most of all, it is found that administration of squalene in combination with DMHCA and interferon beta-1b, strongly reduced EAE disease severity compared to untreated controls and to single and double compound treated animals (Figure 8). This was further supported by reduced number of inflammatory cells (CD4 and CD8 T-cells, CD11b+/CD45high macrophages, CD11b+/CD45low microglia) within spinal cord tissue (Figure 9 a-d).

In one embodiment, the present invention therefore relates to the squalene for use in treatment and/or prophylaxis of demyelinating disease is associated with microglial activation/inflammation and selected from multiple sclerosis (MS), optic neuritis, neuromyelitis optica (Devic's disease), transverse myelitis, acute disseminated encephalomyelitis (ADEM), adrenoleukodystrophy, adrenomyeloneuropathy, acute hemorrhagic leucoencephalitis (AHL), progressive multifocal leukoencephalopathy (PML), central pontine myelinolysis (CPM), extrapontine myelinolysis (EPM), hypoxic-ischemic demyelination, and Alzheimer's disease.

Preferably, the present invention relates to the squalene for use in treatment and/or prophylaxis of demyelinating disease associated with microglial activation/inflammation selected from Alzheimer's disease or multiple sclerosis.

More preferably, the present invention relates to the squalene for use in treatment and/or prophylaxis of multiple sclerosis caused by, and/or associated with inflammation and demyelination in the central nervous system especially in the central nervous system's myelinated nerve fibers.

Squalene is usually obtained from natural sources, in particular, shark liver oil, amaranth oil, olive oil, soybean, hazelnuts oil, peanuts oil, corn oil, and grape seed oil. (O. Popa et al., "Methods for Obtaining and Determination of Squalene from Natural Source", BioMed Research International, 2015, pp.1-16).

Therefore, in one embodiment of the present invention, squalene is preferably obtained from shark liver oil, amaranth oil, olive oil, soybean, hazelnuts oil, peanuts oil, corn oil, and grape seed oil or produced using biotechnological tools.

Optionally, the squalene extract from shark liver, amaranth, olive, soybean, hazelnuts, peanuts, corn, and grape seed may be directly used for treatment and/or prophylaxis of multiple sclerosis, in particular, clinically isolated syndrome (CIS) caused by, and/or associated with inflammation and demyelination in the central nervous system. Moreover it is also possible to administer commercially available oils such as olive oil enriched or fortified with squalene which was added to the commercially available oil. Such squalene extract containing at least 10% weight of squalene, preferably at least 20% weight of squalene, more preferably at least 30% weight of squalene, most preferably at least 50% weight of squalene. The present invention teaches that a combination of squalene with further active agent shows a synergistic effect on the treatment and/or prophylaxis of multiple sclerosis. For example, it was found in the present invention that a combination of squalene and interferon beta-1b shows excellent synergistic effect on the treatment and/or of prophylaxis of multiple sclerosis as shown in Figure 1C.

Optionally, the present invention relates to the squalene for use in treatment and/or prophylaxis of multiple sclerosis in combination with at least one active agent. Preferably, the at least one active agent is selected from the group consisting of an antibody against CD52 or alemtuzumab, an immune modulatory nucleosid analogue or cladribine, an antibody to the alpha subunit of the IL-2 receptor of T cells (CD25) or daclizumab, a dimethyl fumarate, fingolimod, siponimod or other functional modulators of sphingosine-1 phosphate (S1P)-receptors, galtiramer acetate or a polymer of glutamic acid, lysine, alanine and tyrosine or glatiramer, interferon beta-1a (IFNβ-1a), interferon beta-1b (IFNβ-1b), laquinimod, pegylated interferons or peginterferon beta or peginterferon alpha, an anthracenedione molecule or mitoxantrone, an antibody or fragment thereof against alpha-4 integrin or natalizumab, an antibody against CD20 or ocrelizumab, a corticosteroid such as prednisone and an inhibitor of a dihydroorotate dehydrogenase or teriflunomide, an anti-LINGO-1 antibody or opicinumab, a Liver X Receptor (LXR) agonist, or N,N-dimethyl-3β-hydroxycholenamide (DMHCA).

More preferred, the at least one active agent is selected from the group consisting of. cladribine, daclizumab, a dimethyl fumarate, fingolimod, siponimod, galtiramer, galtiramer acetate, interferon beta-1a (IFNβ-1a), interferon beta-1b (IFNβ-1b), laquinimod, peginterferon alpha, peginterferon beta, mitoxantrone, natalizumab, ocrelizumab, prednisone, teriflunomide, opicinumab, and N,N-dimethyl-3β-hydroxycholenamide (DMHCA).
Such active agents are known as various Trademarks and with different application methods are available.
- Injectable medications
   ∘ Avonex ®(interferon beta-1a)
   ∘ Betaseron ® (interferon beta-1b)
   ∘ Copaxone ® (glatiramer acetate)
   ∘ Extavia ® (interferon beta-1b)
   ∘ Glatiramer Acetate Injection ® (glatiramer acetate -generic equivalent of Copaxone 20 mg and 40 mg doses)
   ∘ Glatopa ® (glatiramer acetate - generic equivalent of Copaxone 20mg and 40mg doses)
   ∘ Plegridy ® (peginterferon beta-1a)
   ∘ Rebif® (interferon beta-1a)
- Oral medications
   ∘ Aubagio ® (teriflunomide)
   ∘ Gilenya ® (fingolimod)
   ∘ Tecfidera ® (dimethyl fumarate)
   ∘ Mavenclad ® (cladribine)
   ∘ Mayzent® (siponimod)
   ∘ SAIK-MS (laquinimod)
- Infused medications
   ∘ Lemtrada ® (alemtuzumab)
   ∘ Novantrone ® (mitoxantrone)
   ∘ Ocrevus ® (ocrelizumab)
   ∘ Tysabri ® (natalizumab)
   ∘ Zenapax ® (daclizumab)
   ∘ Temelimab

In one embodiment of the present invention, squalene is used for treatment and/or prophylaxis of multiple sclerosis preferably in combination with the at least one active agent selected from interferon beta-1a (IFNβ-1a), interferon beta-1b (IFNβ-1b), peginterferon beta-1a, and N,N-dimethyl-3β-hydroxycholenamide (DMHCA). Preferred, squalene is used for treatment and/or prophylaxis of multiple sclerosis preferably in combination with interferon beta-1b (IFNβ-1b) and N,N-dimethyl-3β-hydroxycholenamide (DMHCA).

Such interferon beta 1-a/b and pegylated interferon beta-1a (peginterferon beta-1a) are commercially available as follows:
∘ interferon beta-1a: Avonex®, Rebif®
∘ interferon beta-1b: Betaseron ®/ Betaferon ®
∘ peginterferon beta-1a: Plegridy®

In some embodiments, squalene is used for treatment and/or prophylaxis of multiple sclerosis in combination with the above-mentioned at least one active agent, wherein an weight ratio of the squalene and the above-mentioned at least one active agent is in a range of 100,000:1 to 10:1, preferably 50,000:1 to 10:1, more preferably, 20,000:1 to 100:1, most preferably 10,000:1 to 1,000:1.

Furthermore, a wide variety of medications are used to help manage the symptoms of multiple sclerosis. Below are common symptoms of multiple sclerosis and the medications used to treat those symptoms. Therefore, optionally, the squalene is useful in combination with the following active pharmaceutical agent:

### Bladder Problems

### Dysfunction

- Botox (onabotulinumtoxin A)
- DDAVP Nasal Spray (desmopressin)
- Detrol (tolterodine)
- Ditropan (oxybutynin), Ditropan XL
- Enablex (darifenacin)
- Flomax (tamsulosin)
- Hytrin (terazosin)
- Minipress (prazosin)
- Myrbetriq (mirabegron)
- Oxytrol (oxybutynin)
- Pro-Banthine (propantheline)
- Sanctura (trospium chloride)
- Tofranil (imipramine)
- Vesicare (solifenacin succinate)

### Infection

- Bactrim; Septra (sulfamethoxazole)
- Cipro (ciprofloxacin)
- Macrodantim (nitrofurantoin)
- Hiprex (methenamine)
- Pyridium (phenazopyridine)

### Bowel Dysfunction

- Colace (docusate)
- Dulcolax (bisacodyl)
- Enemeez (docusate stool softener laxative)
- Fleet Enema (sodium phosphate)
- Mineral Oil
- Metamucil (psyllium hydrophilic musilloid)
- Phillips Milk of Magnesia (magnesium hydroxide)
- Sani-Supp suppository (gylcerin)

### Depression

- Cymbalta (duloxetine hydrochloride)
- Effexor (velafaxine)
- Paxil (paroxetine)
- Prozac (fluoxetine)
- Wellbutrin (bupropion)
- Zoloft (sertraline)

### Dizziness and Vertigo

- Antivert (meclizine)

### Emotional Changes

- Nuedexta (dextromethorphan + quinidine)

### Fatigue

- Amantadine
- Provigil (modafinil)
- Prozac (fluoxetine)

### Pain & Itching

- Atarax (hydroxyzine)
- Dilantin (phenytoin)
- Elavil (amitriptyline)
- Klonopin (clonazepam)
- Neurontin (gabapentin)
- Pamelor; Aventyl (nortriptyline)
- Tegetrol (carbamazepine)

### Sexual Problems

- Cialis (tadalafil)
- Levitra (vardenafil)
- Papaverine
- MUSE (alprostadil)
- Prostin VR (alprostadil)
- Viagra (sildenafil)

### Spasticity

- Botox (onabotulinumtoxin A)
- Dantrium (dantrolene)
- Gablofen (baclofen [intrathecal])
- Klonopin (clonazepam)
- Lioresal (baclofen)
- Valium (diazepam)
- Zanaflex (tizanidine)

### Tremors

- Laniazid - Nydrazid (isoniazid)
- Klonopin - Rivotril - Syn-Clonazepam (clonazepam)

### Walking (Gait) Difficulties

- Ampyra (dalfampridine)

### Pharmaceutical compositions and use thereof

Another aspect of the present invention is directed to pharmaceutical compositions comprising squalene as active ingredient.

Preferably, a pharmaceutical composition comprises squalene wherein an amount of squalene is in a range of 10 to 99 % weight, preferred 10 to 90 % weight, more preferred 10 to 80 % weight.

Optionally, the pharmaceutical composition may comprise a squalene extract instead of pure squalene. Said squalene extract is preferably obtained from shark liver, amaranth, olive, soybean, hazelnuts, peanuts, corn, and grape seed may be directly used for treatment and/or prophylaxis of multiple sclerosis, in particular, clinically isolated syndrome (CIS). The pharmaceutical composition comprising said squalene extract and an amount of squalene is in a range of 10 to 99 % weight, preferred 10 to 90 %weight, more preferred 10 to 80 % weight. The pharmaceutical composition comprising squalene extract is also useful for treatment and/or prophylaxis of multiple sclerosis.

Preferably, the above-mentioned pharmaceutical composition further comprising at least one active agent selected from the group consisting of alemtuzumab, cladribine, daclizumab, dimethyl fumarate, fingolimod, galtiramer acetate, interferon beta-1a (IFNβ-1a), interferon beta-1b (IFNβ-1b), laquinimod, peginterferon beta-1, mitoxantrone, natalizumab, ocrelizumab, siponimod, and teriflunomide, more preferably, Interferon beta-1a (IFNβ-1a), interferon beta-1b (IFNβ-1b) and peginterferon beta-1a, most preferably), interferon beta-1b (IFNβ-1b). Said pharmaceutical composition comprising squalene and at least one active agent is also useful for treatment and/or prophylaxis of demyelinating disease associated with microglial activation/inflammation selected from multiple sclerosis (MS), optic neuritis, neuromyelitis optica (Devic's disease), transverse myelitis, acute disseminated encephalomyelitis (ADEM), adrenoleukodystrophy and adrenomyeloneuropathy, preferably multiple sclerosis caused by, and/or associated with inflammation and demyelination in the central nervous system especially in the central nervous system's myelinated nerve fibers.

More preferably, said pharmaceutical composition comprises squalene and at above-mentioned least one active agent, wherein a ratio of the squalene and the above-mentioned at least one active agent is an weight ratio of the squalene and the above-mentioned at least one active agent is in a range of 100,000:1 to 10:1, preferably 50,000:1 to 10:1, more preferably, 20,000:1 to 100:1, most preferably 10,000:1 to 1,000:1.

Optionally, said pharmaceutical composition further comprises at least one pharmaceutically acceptable carrier, excipient and/or diluents. The pharmaceutical compositions of the present invention can be prepared in a conventional solid or liquid carrier or diluent and a conventional pharmaceutically-made adjuvant at suitable dosage level in a known way.

The preferred preparations are adapted for oral application. These administration forms include, for example, pills, tablets, film tablets, coated tablets, capsules, powders and deposits.

Said pharmaceutical composition is useful for use in treatment and/or prophylaxis of multiple sclerosis.
The dose of the invention regarding squalene is 0.1 - 1000 mg/kg, preferred, 0.1 - 500 mg/kg, more preferred 0.1 - 200 mg/kg per body weight in a day.
In some embodiments, squalene or pharmaceutical composition is used treatment and/or prophylaxis of multiple sclerosis, wherein 0.1 - 1000 mg/kg, preferred, 0.1 - 500 mg/kg, more preferred 0.1 - 200 mg/kg per body weight, most preferred 0.1 - 15 mg/kg squalene is administered per body weight in a day.

Preferred, the dose of the invention regarding squalene is administered at least 0.1, 0.15, 0.2, 0.25, 0.3, 0.35, 0.4, 0.45, 0.5, 0.55, 0.6, 0.65, 0.7, 0.75, 0.8, 0.85, 0.9, 0.95, 1 .0, 1 .05, 1 .1 , 1 .15, 1 .2, 1 .25, 1 .3, 1 .35, 1 .4, 1 .45, 1 .5, 2, 2.5, 3, 3.5, 4, 4.5, 5, 5.5, 6, 6.5, 7, 7.5, 8, 8.5, 9, 9.5, 10, 10.5, 1 1 , 1 1 .5, 12, 12.5, 13, 13.5, 14, 14.5 or 15 mg/kg squalene per body weight.

More preferred, squalene or pharmaceutical composition is used treatment and/or prophylaxis of multiple sclerosis, wherein 0.5 - 15 mg/kg squalene is administered per body weight in a day. Preferred, the dose of squalene is between 0.5-14, 1-10, 2-10, 1-8, 1-5 or 0.5-2 mg/kg per body weight.

In some embodiment, the invention relates to pharmaceutical composition for use in the treatment and/or prophylaxis of MS, wherein the pharmaceutical composition is formulated as capsule, gel, syrup, emulsion or oil.

In one embodiment, squalene is administered as the only active ingredient in a composition or formulation as discussed above. For example, a composition of squalene may be administered to a MS patient or a perspective patient. The composition of squalene may be combined with other components such as any pharmaceutically acceptable carrier, pharmaceutically acceptable sterile aqueous or nonaqueous solutions, dispersions, suspensions or emulsions, adjuvants such as preservatives, wetting agents, emulsifying agents and dispersing agents, or other necessary substances.

In some embodiments, pharmaceutical composition of the invention is administered by oral, dermal, intradermal, intragastral, intracutan, intravasal, intravenous, intramuscular, intraperitoneal, percutan, rectal, subcutaneous, or transdermal application. Squalene can be applied as nutraceutical as dietary supplement.

The term "pharmaceutically acceptable," as used herein, refers to the compound or composition or carrier being suitable for administration to a subject to achieve the treatments described herein, without unduly deleterious side effects in light of the necessity of the treatment.

The term "therapeutically effective amount" or "pharmaceutically appropriate dosage," as used herein, refers to the amount of the compounds or dosages that will elicit the biological or medical response of a subject, tissue or cell that is being sought by the researcher, veterinarian, medical doctor or other clinician.

As used herein, "pharmaceutically-acceptable carrier" includes any and all dry powder, solvents, dispersion media, coatings, antibacterial and antifungal agents, isotonic agents, absorption delaying agents, and the like. Pharmaceutically-acceptable carriers are materials, useful for the purpose of administering the compounds in the method of the present invention, which are preferably non-toxic, and may be solid, liquid, or gaseous materials, which are otherwise inert and pharmaceutically acceptable, and are compatible with the compounds of the present invention. Examples may include sugars such as, but not limited to, lactose, glucose and sucrose; starches such as, but not limited to, corn starch and potato starch; cellulose and its derivatives such as, but not limited to, sodium carboxymethyl cellulose, ethyl cellulose and cellulose acetate; powdered tragacanth; malt; gelatin; talc; excipients such as, but not limited to, cocoa butter and suppository waxes; oils such as, but not limited to, peanut oil, cottonseed oil, safflower oil, sesame oil, olive oil, corn oil and soybean oil; glycols; such a propylene glycol; esters such as, but not limited to, ethyl oleate and ethyl laurate; agar; buffering agents such as, but not limited to, magnesium hydroxide and aluminum hydroxide; alginic acid; pyrogen-free water; isotonic saline; Ringer's solution; ethyl alcohol, and phosphate buffer solutions, as well as other non-toxic compatible lubricants such as, but not limited to, sodium lauryl sulfate and magnesium stearate, as well as coloring agents, releasing agents, coating agents, sweetening, flavoring and perfuming agents, preservatives and antioxidants may also be present in the composition. Further examples of such carriers include, various lactose, mannitol, oils such as com oil, buffers such as PBS, saline, polyethylene glycol, glycerin, polypropylene glycol, dimethylsulfoxide, an amide such as dimethylacetamide, a protein such as albumin, and a detergent such as Tween 80, mono- and oligopolysaccharides such as glucose, lactose, cyclodextrins and starch.

The term "administering" or "administration," as used herein, refers to providing the squalene or pharmaceutical composition of the invention to a subject suffering from or at risk of the diseases or conditions to be treated or prevented.

The term "systemic delivery," as used herein, refers to any suitable administration methods which may delivery the compounds in the present invention systemically. In one embodiment, systemic delivery may be selected from the group consisting of oral, parenteral, intranasal, inhaler, sublingual, rectal, intracisternal, and transdermal, intravaginal, intraperitoneal, topically (as by powders, ointments or drops), bucal or as an oral or nasal spray administrations. The term "parenterally" as used herein, refers to modes of administration which include intravenous, intramuscular, intraperitoneal, intrasternal, subcutaneous and intraarticular injection and infusion.

The pharmaceutical compositions according to the present invention containing squalene or squalene extract as active ingredient will typically be administered together with suitable carrier materials selected with respect to the intended form of administration, i.e. for oral administration in the form of tablets, capsules (either solid filled, semi-solid filled or liquid filled), powders for constitution, extrudates, deposits, gels, elixirs, dispersible granules, syrups, suspensions, and the like, and consistent with conventional pharmaceutical practices. For example, for oral administration in the form of tablets or capsules, the active drug component may be combined with any oral non-toxic pharmaceutically acceptable carrier, preferably with an inert carrier like lactose, starch, sucrose, cellulose, magnesium stearate, dicalcium phosphate, calcium sulfate, talc, mannitol, ethyl alcohol (liquid filled capsules) and the like. Moreover, suitable binders, lubricants, disintegrating agents and coloring agents may also be incorporated into the tablet or capsule. Powders and tablets may contain about 5 to about 95 weight % of the stilbenoid derived compound and/or the respective pharmaceutically active salt as active ingredient.

Suitable binders include starch, gelatine, natural sugars, and corn sweeteners, natural and synthetic gums such as acacia, sodium alginate, carboxymethylcellulose, polyethylene glycol and waxes. Among suitable lubricants there may be mentioned boric acid, sodium benzoate, sodium acetate, sodium chloride, and the like. Suitable disintegrants include starch, methylcellulose, guar gum, and the like. Sweetening and flavouring agents as well as preservatives may also be included, where appropriate. The disintegrants, diluents, lubricants, binders etc. are discussed in more detail below.

Moreover, the pharmaceutical compositions of the present invention may be formulated in sustained release form to provide the rate controlled release of any one or more of the components or active ingredients to optimise the therapeutic effect(s), e.g. antihistaminic activity and the like. Suitable dosage forms for sustained release include tablets having layers of varying disintegration rates or controlled release polymeric matrices impregnated with the active components and shaped in tablet form or capsules containing such impregnated or encapsulated porous polymeric matrices.

Liquid form preparations include solutions, suspensions, and emulsions. As an example, there may be mentioned water or water/propylene glycol solutions for parenteral injections or addition of sweeteners and opacifiers for oral solutions, suspensions, and emulsions. Liquid form preparations may also include solutions for intranasal administration. Aerosol preparations suitable for inhalation may include solutions and solids in powder form, which may be present in combination with a pharmaceutically acceptable carrier such as an inert, compressed gas, e.g. nitrogen. For preparing suppositories, a low melting fat or wax, such as a mixture of fatty acid glycerides like cocoa butter is melted first, and the active ingredient is then dispersed homogeneously therein e.g. by stirring. The molten, homogeneous mixture is then poured into conveniently sized moulds, allowed to cool, and thereby solidified.

Also included are solid form preparations which are intended to be converted, shortly before use, to liquid form preparations for either oral or parenteral administration. Such liquid forms include solutions, suspensions, and emulsions.

The transdermal compositions may have the form of a cream, a lotion, an aerosol and/or an emulsion and may be included in a transdermal patch of the matrix or reservoir type as is known in the art for this purpose.

The term capsule as recited herein refers to a specific container or enclosure made e.g. of methyl cellulose, polyvinyl alcohols, or denatured gelatines or starch for holding or containing compositions comprising the active ingredient(s).

Capsules with hard shells are typically made of blended of relatively high gel strength gelatines from bones or pork skin. The capsule itself may contain small amounts of dyes, opaquing agents, plasticisers and/or preservatives. Under tablet a compressed or moulded solid dosage form is understood which comprises the active ingredients with suitable diluents. The tablet may be prepared by compression of mixtures or granulations obtained by wet granulation, dry granulation, or by compaction well known to a person of ordinary skill in the art.

Oral gels refer to the active ingredients dispersed or solubilised in a hydrophilic semi-solid matrix. Powders for constitution refers to powder blends containing the active ingredients and suitable diluents which can be suspended e.g. in water or in juice.

Suitable diluents are substances that usually make up the major portion of the composition or dosage form. Suitable diluents include oils, sugars such as lactose, sucrose, mannitol, and sorbitol, starches derived from wheat, corn rice, and potato, and celluloses such as microcrystalline cellulose. The amount of diluent in the composition can range from about 5 to about 95 % by weight of the total composition, preferably from about 25 to about 75 weight %, and more preferably from about 30 to about 60 weight %.

The term disintegrants refers to materials added to the composition to support break apart (disintegrate) and release the pharmaceutically active ingredients of a medicament. Suitable disintegrants include starches, "cold water soluble" modified starches such as sodium carboxymethyl starch, natural and synthetic gums such as locust bean, karaya, guar, tragacanth and agar, cellulose derivatives such as methylcellulose and sodium carboxymethylcellulose, microcrystalline celluloses, and cross-linked microcrystalline celluloses such as sodium croscaramellose, alginates such as alginic acid and sodium alginate, clays such as bentonites, and effervescent mixtures. The amount of disintegrant in the composition may range from about 2 to about 20 weight % of the composition, more preferably from about 5 to about 10 weight %.

Binders are substances which bind or "glue" together powder particles and make them cohesive by forming granules, thus serving as the "adhesive" in the formulation. Binders add cohesive strength already available in the diluent or bulking agent. Suitable binders include sugars such as sucrose, starches derived from wheat corn rice and potato, natural gums such as acacia, gelatine and tragacanth, derivatives of seaweed such as alginic acid, sodium alginate and ammonium calcium alginate, cellulose materials such as methylcellulose, sodium carboxymethylcellulose and hydroxypropylmethylcellulose, polyvinylpyrrolidone, and inorganic compounds such as magnesium aluminium silicate. The amount of binder in the composition may range from about 2 to about 20 weight % of the composition, preferably from about 3 to about 10 weight %, and more preferably from about 3 to about 6 weight %.

Lubricants refer to a class of substances which are added to the dosage form to enable the tablet granules etc. after being compressed to release from the mould or die by reducing friction or wear. Suitable lubricants include metallic stearates such as magnesium stearate, calcium stearate, or potassium stearate, stearic acid, high melting point waxes, and other water soluble lubricants such as sodium chloride, sodium benzoate, sodium acetate, sodium oleate, polyethylene glycols and D,L-leucine. Lubricants are usually added at the very last step before compression, since they must be present at the surface of the granules. The amount of lubricant in the composition may range from about 0.2 to about 5 weight % of the composition, preferably from about 0.5 to about 2 weight %, and more preferably from about 0.3 to about 1.5 weight % of the composition.

Glidents are materials that prevent caking of the components of the pharmaceutical composition and improve the flow characteristics of granulate so that flow is smooth and uniform. Suitable glidents include silicon dioxide and talc. The amount of glident in the composition may range from about 0.1 to about 5 weight % of the final composition, preferably from about 0.5 to about 2 weight %.

Colouring agents are excipients that provide coloration to the composition or the dosage form. Such excipients can include food grade dyes adsorbed onto a suitable adsorbent such as clay or aluminium oxide. The amount of the colouring agent may vary from about 0.1 to about 5 weight % of the composition, preferably from about 0.1 to about 1 weight %.

### Description of the figures:

**Figure 1****: Clinical score of MOG35-55 induced EAE animals**
   a) Mean clinical score ± SEM of mice in the prophylactic squalene treatment paradigm. Mice received squalene diet commencing at 14 days before immunization.
   b) Mean clinical score ± SEM of mice in the therapeutic squalene treatment paradigm. Mice received squalene diet commencing at the day of disease onset.
   c) Mean clinical score ± SEM of mice in the prophylactic squalene treatment paradigm in combination with IFNβ1b. IFNβ1b was given as daily i.p. injections starting from day 3 after EAE induction. The number of mice in each experimental group is given in parentheses.
**Figure 2****: Squalene induced reduction of inflammatory cells in spinal cord tissue**
   a) Serum squalene concentration measured by FPLC 28 days post immunization (Prophylactic squalene treatment) measured by FPLC.
   b-d) Number of b) CD45+/CD11b+ myeloid cells c) abTCR/CD4+, d) abTCR/CD8+ and per gram spinal cord measured by flow cytometry at the peak of EAE disease (day 16) after therapeutic squalene administration in comparison to mice that received normal chow (Ctrl). Bars represent means with individual data points (n= 6 mice per group, Student's t-test, *P<0.05).
**Figure 3****: No impact of squalene on peripheral inflammatory cell number**
   a-c) Number of a) abTCR/CD4+, b) abTCR/CD8+ and c) CD45+/CD11b+ myeloid cells per ml collected blood measured by flow cytometry at peak of EAE disease (day 16) after therapeutic squalene administration as described in Fig. 2 were comparable in both treatment groups.
**Figure 4****: Squalene induced spinal cord tissue expression profile**
   a-b) RT-qPCR expression profile of a) cellular marker genes and cholesterol synthesis / metabolism genes and b) inflammatory genes in spinal cord lysates of mice fed normal chow or chow supplemented with squalene in remission phase (prophylactic treatment paradigm). Bars represent normalized means (n=6 animals) of fold expression ± SEM (set to 1) (Student's t-test, *P<0.05, **P<0.01, ***P<0.001).
**Figure 5****: Squalene induced expression profile in isolated CD11b+ myeloid cells**
   a) RT-qPCR expression profile of inflammatory genes in MACS isolated CD11b* myeloid cells during acute phase (15 days post immunization) phase after prophylactic squalene administration. Bars represent normalized means (n=6 animals) ± SEM (set to 1) (Student's t-test, *P<0.05, **P<0.01, ***P<0.001).
   b) Expression of cholesterol export genes *Abca1* and *Apoe* in MACS isolated CD11b* myeloid cells as in a) during acute phase. Bars represent means normalized to untreated control mice (Student's t-test, *P<0.05)
**Figure 6****: Squalene induced remyelination in lysolecithin induced lesions**
   a) Representative images of spinal cord sections 14 days post lesion (dpl) with 1µl 1% lysolecithin stained for the oligodendrocyte marker CAII. Squalene treatment started at day of lesion induction. Dotted line marks lesion area with increased cellular density (DAPI). Scale = 100µm
   b) Quantification of CAII+ oligodendrocytes within lesions shown in a). Bars represent means of control (n=5) and squalene (n=6) treated animals. Significance evaluated by Student's t-test, **P<0.01.
   c) Quantification of lesion area depicted in a). Bars represent means of control (n=5) and squalene (n=6) treated animals.
   d) Serum squalene concentration measured by FPLC of animals (n=3) analyzed in a-c).
   e) Representative images of spinal cord sections as in a) stained for MBP labeling myelin.
   f) Quantification of MBP+ myelin within lesions shown in e).

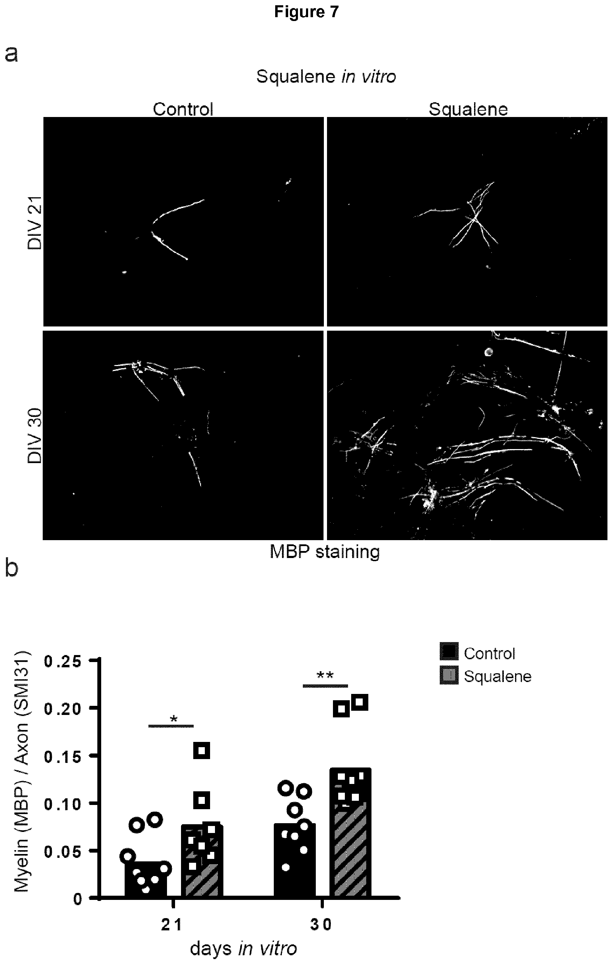

**Figure 7****: Squalene support myelination *in vitro***
   a) Representative images of myelinating co-culture at 21 days in vitro (DIV) and 30 DIV treated with or without 100 µM squalene stained for myelinated axon segments (MBP).
   b) Quantification of MBP positive area normalized to SMI31 positive axons at 21DIV and 30 DIV. Bars represent means of individual control and squalene treated cultures (n=4 biological replicates). Significance evaluated by 1way ANOVA following multiple comparison correction with Holm-Sidak method, **P<0.01.
**Figure 8****: Clinical score of EAE induced animals treated with squalene combination drug therapy**
   a) Mean clinical score ± SEM of mice treated according to the therapeutic treatment paradigm with or without squalene, the synthetic LXR agonist DMHCA, and interferon. Mice received squalene and DMHCA supplemented diet commencing from the day of disease onset. IFNβ1b was given daily by i.p. injections starting from day 3 after induction of the EAE.
   b) Expression of cholesterol export genes in MACS isolated CD11b* myeloid cells during chronic phase from DMHCA treated animals a). Bars represent means normalized to untreated control mice (Student's t-test, **P<0.01, ***P<0.001).
**Figure 9****: Squalene induced reduction of inflammatory cells in combination with anti-inflammatory therapy**
   a-d) Number of a) abTCR/CD4+, b) abTCR/CD8+ c) CD11b+/CD45^{high} macrophages and d) CD11b+/CD45^{low} microglia per gram spinal cord from mice treated as in Fig. 7, measured by flow cytometry. Bars represent means with individual data points (Student's t-test, *P<0.05, **P<0.01).

The following examples are included to demonstrate preferred embodiments of the invention. It should be appreciated by those of skill in the art that the techniques disclosed in the examples which follow represent techniques discovered by the inventor to function well in the practice of the invention, and thus can be considered to constitute preferred modes for its practice. However, those of skill in the art should, in light of the present disclosure, appreciate that many changes can be made in the specific embodiments which are disclosed and still obtain a like or similar result without departing from the spirit and scope of the invention.

Further modifications and alternative embodiments of various aspects of the invention will be apparent to those skilled in the art in view of this description. Accordingly, this description is to be construed as illustrative only and is for the purpose of teaching those skilled in the art the general manner of carrying out the invention. It is to be understood that the forms of the invention shown and described herein are to be taken as examples of embodiments. Elements and materials may be substituted for those illustrated and described herein, parts and processes may be reversed, and certain features of the invention may be utilized independently, all as would be apparent to one skilled in the art after having the benefit of this description of the invention. Changes may be made in the elements described herein without departing from the spirit and scope of the invention as described in the following claims.

### EXAMPLES

### Methods

### Mice

For MOG-EAE, mice purchased from Charles River were immunized subcutaneously with 200 mg myelin oligodendrocyte glycoprotein peptide 35-55 (MOG35-55) in complete Freund's adjuvant (M. tuberculosis at 3.75 mg ml⁻¹) and i.p. injected twice with 500 ng pertussis toxin. Animals were examined daily and scored for clinical signs of the disease. If disease did not start within 15 days after induction or the clinical score rose above 4, animals were excluded from the analysis. The clinical score was: 0 normal; 0.5 loss of tail tip tone; 1 loss of tail tone; 1.5 ataxia, mild walking deficits (slip off the grid); 2 mild hind limb weakness, severe gait ataxia, twist of the tail causes rotation of the whole body; 2.5 moderate hind limb weakness, cannot grip the grid with hind paw, but able to stay on a upright tilted grid; 3 mild paraparesis, falls down from a upright tiled grid; 3.5 paraparesis of hind limbs (legs strongly affected, but move clearly); 4 paralysis of hind limbs, weakness in forelimbs; 4.5 forelimbs paralyzed; 5 moribund/dead. Mice received 0.5% [v/w] squalene (Sigma) chow commencing either two weeks before immunization defined as prophylactic regimen or at the first appearance of EAE symptoms defined as therapeutic regimen and continued until day 28. DMHCA (Avanti) was dissolved in corn oil (Sigma) and given therapeutically in chow at a dosage of 8 mg kg⁻¹. IFNβ1b (Bayer) was administrated by daily intraperitoneal (i.p.) injections 30.000 U per animal.

Focal spinal cord demyelinating lesions were induced under MMF anesthesia (1.0 mg kg⁻¹ midazolam, 0.05 mg kg⁻¹ medetomidine and 0.02 mg kg⁻¹ fentanyl) by stereotactic injection of 1 µl lysolecithin (1%, from egg yolk, alpha-lysophosphatidyl-choline, Sigma) into the left and right ventro-lateral funiculus at Th10 of 8-week old animals. The injection was performed at a rate of ∼1 µl min⁻¹. This procedure created fusiform demyelinating lesions, 5-6 mm in length. At the day of injection, mice were randomly assigned to normal or 0.5% [v/w] squalene supplemented chow for 14 days, after which the animals were killed, and the spinal cord processed for histology. For serum isolation, blood was collected by cardiac puncture, and serum was prepared after 4h clotting by centrifugation.

### Cell isolation and flow cytometry.

Single-cell suspensions from spinal cords were obtained via mechanical dissociation on a cell strainer. Immune cells were separated over a two-phase Percoll-density gradient (70% / 30%). Blood was collected by cardiac puncture in EDTA (80mM) and single-cell suspension was obtained by centrifugation over lymphocyte separation medium (LSM 1077, PAA). Staining of abTCR/CD4 T cells, abTCR/CD8 T cells and CD45/CD11b cells (macrophages/microglia) was performed using the following antibodies in a 1:200 dilution: Anti-CD3e (clone 145-2C11), BioLegend; anti-CD4 (clone GK 1.5), BD; anti-CD8 (clone 53-6.7), BD; anti-CD8 (clone 53-6.7), BD; anti-CD11b (clone M1/70), BioLegend; anti-CD45.2 (clone 104), BioLegend. The addition of Calibrite APC beads (BD) allowed for cell quantification. Flow cytometry was performed using a FACSCalibur operated by Cell Quest software (Becton Dickinson).

### Magnetic cell isolation.

Microglia/Macrophages were isolated according to the adult brain dissociation protocol (Miltenyi biotec). Spinal cord was isolated on ice followed by removal of meninges. Antibody labeling steps were done according to the respective antibody Microbead kit protocol (Miltenyi biotec), oligodendrocytes (O4, 130-096-670) and microglia (CD11b, 130-093-636). Purity of cell populations was routinely determined by qPCR on extracted and reverse transcribed RNA (see below) and revealed only minimal contamination by other cell types.

### Expression analyses.

For tissue expression analyses, spinal cord tissue was dissected from ∼Th10 - ∼ L5. RNA was isolated using QIAshredder and RNeasy protocols (Qiagen). Concentration and quality of RNA was evaluated using a NanoDrop spectrophotometer and RNA Nano (Agilent). cDNA was synthesized with Superscript III (Invitrogen) and quantitative PCRs were done in triplicates with the GoTaq qPCR Master Mix (Promega) on a 7500 Fast Real- Time PCR System (Applied Biosystems). Expression values were normalized to the mean of three - five housekeeping genes, HPRT (Hypoxanthin-Phosphoribosyl-Transferase 1) and Rplp0 (60S acidic ribosomal protein P), Rps13 (Ribosomal Protein S13), Gapdh (Glyceraldehyde-3-Phosphate Dehydrogenase), 18S (18S ribosomal RNA) and quantification was done by applying the ΔΔCt method, normalized to age matched untreated controls (set to 1). All primers were intron-spanning.

### Histochemistry.

Mice were perfused with 4% paraformaldehyde (PFA). Spinal cord tissue was postfixed overnight, embedded in paraffin and cut into 5 mm sections (HMP 110, MICROM). For immunohistological analyses, sections were deparaffinized followed by antigen-retrieval in sodium citrate buffer (0.01 M, pH 6.0). For immunofluorescence, sections were blocked with serum free protein block (Dako). Primary antibodies were diluted in 2% bovine serum albumin (BSA)/PBS and incubated for 48 h followed by fluorophor coupled secondary antibodies. Lesion area (DAPI clustered nuclei), oligodendrocyte cell number (CAII, carbonic anhydrase 2) and myelin positive area (MBP, myelin basic protein) were evaluated.

### Fast-Protein Liquid Chromatography

Lipids were extracted from serum samples according to Bligh and Dyer (cit. Can. J. Biochem, 1959). The chloroform phase containing the lipids was evaporated at 40°C in a vacuum concentrator (Qiagen) and extracted lipids dissolved and saponified in glass tubes with 0.5 M KOH in EtOH for 30 min. Non-saponifiable lipids were extracted with n-hexane. After evaporation of the n-hexane phase at 40°C in a vacuum concentrator, non-saponifiable lipids were dissolved in MetOH and subjected to reverse phase HPLC (250/4 Nucleoshell RP C18 (Macherey and Nagel) connected to an Äkta FPLC (GE Healthcare), flow rate 0.25 ml/min, liquid phase acetonitril:EtOH 70:30 (v/v), online UV detection at 205. Squalene retention and peak amplitude were related to injections of a range of concentration standards in MetOH. Amplitudes were normalized to the neutral lipid recovery as assessed via online detection of fluorescence of dehydroergosterol (excitation 325 nm, emission 375 nm, Quantamaster (PTI/Horiba) that was spiked into the serum sample.

### Cell culture

Myelinating co-cultures were established as described (Thomson, C. E. *et al.* 2008) with minor modifications. Briefly, E13 embryonic spinal cords were digested in 0.125% Trypsin solution in HBSS (without ^{Ca+2} and Mg⁺²) at 37 °C for 20 min. After stopping the digestion with 1ml plating media (DMEM, 25% horse serum,25% HBSS, 50 mg ml⁻¹ DNAse) the tissue was homogenized by gentle trituration and centrifuged for 5min. 150,000 cells were plated per poly-L-lysine coated coverslip; 3 coverslips per 35mm Petri dish. After cell attachment in plating media, differentiation media was added (low glucose DMEM, 10 mg ml⁻¹ insulin, 10 ng ml⁻¹ biotin, 50nM hydrocortisone, 0.5% N1-mix). N1 mix was 1mg ml⁻¹ apo-transferrin, 20mM putrescine, 4 mM progesterone, and 6 mM sodium selenite. 50% media change was performed every 24-48 h with differentiation media. Squalene (100 µM) was added at day 3 to cultures. After 12 days, insulin was removed from differentiation media. Coverslips were fixed with PFA after 21 and 30 days in culture and permeabilized with -20 °C methanol for 10 min. Axonal (SMI31) area and the area with myelin sheaths (MBP) of seven randomly chosen visual fields of myelinating co-cultures (x 20 magnification) was measured by automated threshold with Fiji Software (SMI31 Otsu, MBP Triangle). Specimens were analyzed on an Axiophot observer.Z1 (Zeiss) equipped with an AxioCam MRm and the ZEN 2012 blue edition software and evaluated with Image J software.

### Statistical analyses.

Statistical evaluation was done by unpaired Student's t-test for pairwise comparisons or by ANOVA for comparisons of more than two groups. For all statistical tests, significance was measured against an alpha value of 0.05. All error bars show s.e.m. P values are shown as *P<0.05; **P<0.01; ***P<0.001. Data analysis was performed blind to the experimental groups.

### Results

Here, the applicant could show that the natural triterpene squalene that is an intermediate of cholesterol biosynthesis has the potential to ameliorate disease severity in a mouse model of immune mediated myelin disease (EAE) in a prophylactic (Figure 1a) and therapeutic (Figure 1b) treatment paradigm. Animals showed lower clinical scores and did not reach clinical symptoms/scores even at peak of disease (-day 18), suggesting robust anti-inflammatory effects. Importantly, in addition to the already marked single compound effect (Fig 1a-b), squalene administration further reduced disease severity in a combined treatment with daily injections of the first line standard drug in Multiple Sclerosis, interferon beta-1b (Figure 1c). These data suggest that squalene and interferon target different disease processes. Because of this add-on benefit, there is a clear rationale to consider squalene supplementation or a derivative as a future therapy for inflammatory myelin diseases.

Dietary squalene administration leads to a marked increase in serum squalene in the two disease models tested (Figure 2a, 6e). Under physiological conditions, squalene is not transported from the circulation across the blood-brain barrier to the CNS. Considering blood-brain barrier breach EAE already before the onset of clinical symptoms (Davalos et al., Annals of Neurology, 2014, 75, pp.303-308; Schellenberg et al., Magnetic Resonance in Medicine, 2007, 58, pp.298-305.) and locally in lysolecithin-induced lesions, our data is compatible with the possibility of increased compound availability in the CNS parenchyma.

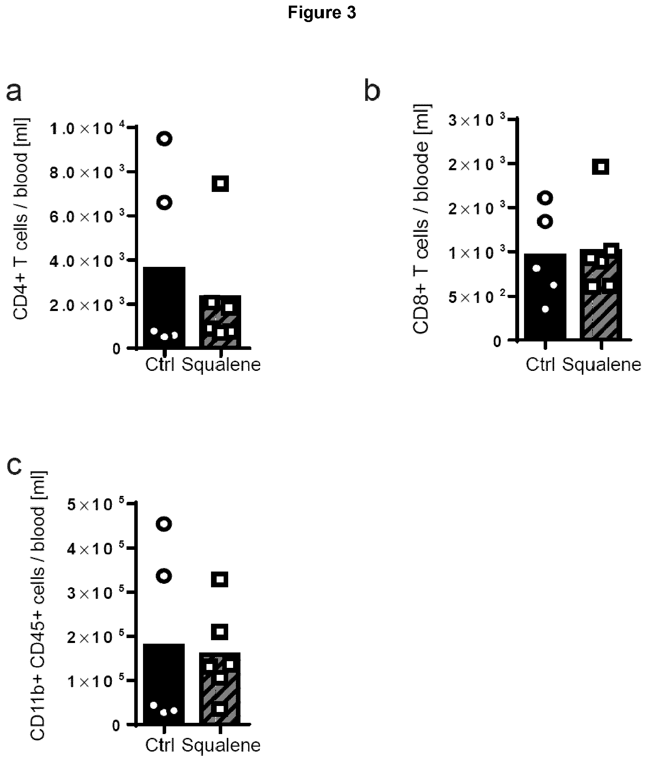

By FACS analysis of spinal cord tissue from squalene treated animals and untreated controls, we did not observe differences in the number of peripheral immune cells (Figure 3 a-c) suggesting comparable T cell priming in peripheral lymphoid organs. However, we cannot exclude anti-inflammatory effects of squalene on peripheral inflammatory cells e.g. macrophages. In contrast, we found reduced numbers of inflammatory CD4+ and CD8+ T cells as well as CD11b+/CD45+ myeloid cells at peak of disease (Figure 2 b-d). These data indicate that squalene therapy leads to a reduction of recruited inflammatory cells into the spinal cord parenchyma and likely an amelioration of the inflammatory milieu within the CNS.

By targeted expression profiling in spinal cord of EAE treated mice, we found significantly increased expression of genes related to myelination and oligodendrocyte differentiation (Plp1, Car2, Olig2, Pdgfra, Cspg4), as well as cholesterol synthesis (Hmgc1, Hmgcr, Fdft1, Dhcr24, Cyp51a1, Mvk), cholesterol export (Abca1, Abcg1, Abcg4, Apoe, Clu) and cholesterol uptake (Ldlr, Vldlr, Apobr, Lrp1, Lrp2) in squalene supplemented mice compared to chow fed controls (Figure 4a). As cholesterol is rate limiting for myelin membrane synthesis (Saher et al., (2005) Nat. Neuro 8 (4): 468-475), together these data show that squalene facilitates remyelination. We speculate that increased squalene availability induced remyelination in EAE lesions by supporting endogenous cholesterol synthesis and secondarily transport. In addition, we detected reduced expression of pro-inflammatory mediator genes while gene expression of the anti-inflammatory molecules 1110 and Tgfb1 was significantly increased (Figure 4b). This shift of inflammatory mediators towards an anti-inflammatory milieu suggests an environment permissive for remyelination.

To directly test the inflammatory status of microglia/macrophages, we performed targeted expression profiling on acutely isolated CD11b+ cells (Figure 5). Indeed, isolated microglia/macrophages from squalene treated mice showed reduced expression levels of pro-inflammatory mediator genes such as TNF and IL1β and increased expression of the anti-inflammatory molecules such as TGFβ1 compared to the same cell fraction from chow fed mice (Figure 5a). In addition, microglia/macrophages from squalene treated mice upregulated the major cholesterol export genes Abca1 and Apoe (Figure 5b) implying increased delivery of microglial cholesterol to oligodendrocytes for remyelination. Together, these data demonstrate that dietary squalene supplementation has a direct impact on tissue microglia/macrophages during EAE, inducing a shift to an anti-inflammatory phenotype and increased cholesterol efflux that both supports tissue repair. We speculate that mechanistically squalene particularly increases LXR signaling directly or via desmosterol that facilitated the export of cholesterol.

Oligodendrocyte cholesterol is rate-limiting for myelin biogenesis. Squalene as an intermediate of cholesterol biosynthesis could fuel into the cholesterol biosynthesis pathway in oligodendrocytes to support remyelination. Therefore, we used the lysolecithin demyelination model that lacks an inflammatory component and evaluated oligodendrocyte differentiation and remyelination in squalene supplemented mice. The number of CAII positive mature oligodendrocytes and the degree of MBP positive myelinated axons within lesion area was strongly increased in squalene supplemented mice compared to control mice (Figure 6a-f) while the size of the lesion area determined by DAPI positive increased nuclear density was comparable (Figure 6c). To test, weather squalene directly supports oligodendrocyte differentiation and myelination, in vitro studies were performed. Myelinating cultures showing spontaneous oligodendrocyte myelination, starting around 18 days in vitro were treated with or without squalene and myelin area (MBP) was quantified in relation to axonal density (SMI31). We observed increased MBP positive area (Figure 7a-b) at 21 days in vitro (DIV) and 30 DIV indicating a direct effect of squalene availability on myelination.

In addition to mediating an anti-inflammatory phenotype in microglia/macrophages in EAE (Fig. 4b, 5a) and to facilitating myelination by oligodendrocytes (Fig. 6 , Fig 7), squalene treatment enhance the expression of cholesterol efflux genes that likely facilitated remyelination by oligodendrocytes (Fig. 5b). Cholesterol efflux and attenuated inflammation is the major outcome of LXR signaling (J. R. Secor McVoy et al., Journal of Neuroinflammation, 2015, 12:27; D. G. Thomas et al., Cell Reports, 2018, 25, pp.3774-3785). By inducing LXR signaling with the LXR agonist N,N-dimethyl-3β-hydroxycholenamide (DMHCA) in microglia/macrophages, EAE disease severity could be reduced (Figure 8a, Figure 9a-d) paralleled by upregulation of cholesterol efflux genes (Figure 8b). To test, whether squalene administration could potentiate the efficiency of anti-inflammatory therapy due to its pro-remyelinating on oligodendrocytes and inflammation dampening effect on microglia/macrophages, we combined squalene therapy with DMHCA. In agreement with our hypothesis, squalene supplementation combined with the administration of DMHCA and interferon beta-1b, strongly reduced EAE disease severity compared to untreated controls and to single and double compound treated animals (Figure 8). This was further supported by reduced number of inflammatory cells (CD4 and CD8 T-cells, CD11b+/CD45high macrophages, CD11b+/CD45low microglia) within spinal cord tissue (Figure 9 a-d).

## Claims

1. Squalene for use in treatment and/or prophylaxis of demyelinating disorders/diseases selected from the group consisting of:
Lysosomes and Lysosomal Disorders, Metachromatic Leukodystrophy, Multiple Sulfatase Deficiency, Globoid Cell Leukodystrophy (Krabbe Disease), GM1 Gangliosidosis, GM2 Gangliosidosis, Fabry Disease, Fucosidosis, Mucopolysaccharidoses, Free Sialic Acid Storage Disorder, Neuronal Ceroid Lipofuscinoses, Adult Polyglucosan Body Disease, Peroxisomes and Peroxisomal Disorders, Peroxisome Biogenesis Defects, Peroxisomal D-Bifunctional Protein Deficiency, Peroxisomal Acyl-CoA Oxidase Deficiency, X-linked Adrenoleukodystrophy, Refsum Disease, Mitochondria and Mitochondrial Disorders, Mitochondrial Encephalopathy with Lactic Acidosis and Stroke-like Episodes, Leber Hereditary Optic Neuropathy, Kearns-Sayre Syndrome, Mitochondrial Neurogastrointestinal Encephalomyopathy, Leigh Syndrome and Mitochondrial Leukoencephalopathies, Pyruvate Carboxylase Deficiency, Multiple Carboxylase Deficiency, Cerebrotendinous Xanthomatosis, Cockayne Syndrome, Trichothiodystrophy with Photosensitivity, Pelizaeus-Merzbacher Disease and X-linked Spastic Paraplegia Type 2, 18q- Syndrome, Phenylketonuria, Glutaric Aciduria Type 1, Propionic Acidemia, Nonketotic Hyperglycinemia, Maple Syrup Urine Disease, 3-Hydroxy 3-Methylglutaryl-CoA Lyase Deficiency, Canavan Disease, L-2-Hydroxyglutaric Aciduria, D-2-Hydroxyglutaric Aciduria, Hyperhomocysteinemias, Urea Cycle Defects, Serine Synthesis Defect Caused by 3-Phosphoglycerate Dehydrogenase Deficiency, Molybdenum Cofactor Deficiency and Isolated Sulfite Oxidase Deficiency, Galactosemia, Sjogren-Larsson Syndrome, Lowe Syndrome, Wilson Disease, Menkes Disease, Fragile X Premutation, Hypomelanosis of Ito, Incontinentia Pigmenti, Alexander Disease, Giant Axonal Neuropathy, Megalencephalic Leukoencephalopathy with Subcortical Cysts, Congenital Muscular Dystrophies, Myotonic Dystrophy Type , Myotonic Dystrophy Type 2, X-linked Charcot-Marie-Tooth Disease, Oculodentodigital Dysplasia, Leukoencephalopathy with Vanishing White Matter, Aicardi-Goutières Syndrome, Leukoencephalopathy with Calcifications and Cysts, Leukoencephalopathy with Brain Stem and Spinal Cord Involvement and Elevated White Matter Lactate, Hypomyelination with Atrophy of the Basal Ganglia and Cerebellum, Hereditary Diffuse Leukoencephalopathy with Neuroaxonal Spheroids, Dentatorubropallidoluysian Atrophy, Cerebral Amyloid Angiopathy, Cerebral Autosomal Dominant Arteriopathy with Subcortical Infarcts and Leukoencephalopathy, Cerebral Autosomal Recessive Arteriopathy with Subcortical Infarcts and Leukoencephalopathy, Polycystic Lipomembranous Osteodysplasia with Sclerosing Leukoencephalopathy (Nasu-Hakola Disease), Pigmentary Orthochromatic Leukodystrophy, Adult-Onset Autosomal Dominant Leukoencephalopathies, Inflammatory and Infectious Disorders, Multiple Sclerosis, Optic neuritis, Neuromyelitis optica, Transverse myelitis, Acute Disseminated Encephalomyelitis, Acute hemorrhagic leucoencephalitisAdrenoleukodystrophy and adrenomyeloneuropathy, Progressive Multifocal Leukoencephalopathy, Central Pontine and Extrapontine Myelinolysis, Hypoxic-ischemic demyelination, Alzheimre's disease (AD), Acute Hemorrhagic Encephalomyelitis, Acquired Immunodeficiency Syndrome, Brucellosis, Subacute Sclerosing Panencephalitis, Congenital and Perinatal Cytomegalovirus Infection, Whipple Disease, Toxic Encephalopathies, Iatrogenic Toxic Encephalopathies, Hypernatremia, Marchiafava-Bignami Syndrome, Posterior Reversible Encephalopathy Syndrome, Langerhans Cell Histiocytosis, Post-Hypoxic-Ischemic Damage, Post-Hypoxic-Ischemic Leukoencephalopathy of Neonates, Neonatal Hypoglycemia, Delayed Posthypoxic Leukoencephalopathy, White Matter Lesions of the Elderly Subcortical Arteriosclerotic Encephalopathy, Vasculitis, Leukoencephalopathy and Dural Venous Fistula, Leukoencephalopathy after Chemotherapy and/or Radiotherapy, Gliomatosis Cerebri, Diffuse Axonal Injury, Wallerian Degeneration and Myelin Loss Secondary to Neuronal and Axonal Degeneration.

2. Squalene for use according to claim 1, wherein demyelinating disease is is associated with microglial activation/inflammation and selected from multiple sclerosis (MS), optic neuritis, neuromyelitis optica (Devic's disease), transverse myelitis, acute disseminated encephalomyelitis (ADEM), adrenoleukodystrophy adrenomyeloneuropathy, acute hemorrhagic leucoencephalitis (AHL), progressive multifocal leukoencephalopathy (PML), central pontine myelinolysis (CPM), extrapontine myelinolysis (EPM), hypoxic-ischemic demyelination, and Alzheimer's disease.

3. Squalene for use according to claim 1 or 2, wherein the squalene is obtained from shark liver oil, amaranth oil, olive oil, soybean, hazelnuts oil, peanuts oil, corn oil, and grape seed oil or synthesized using biotechnological tools.

4. Squalene for use according to any one of claims 1 to 3, in combination with at least one active agent selected from the group consisting of alemtuzumab, cladribine, daclizumab, dimethyl fumarate, fingolimod, galtiramer acetate, interferon beta-1a, interferon beta-1b, laquinimod, peginterferon beta-1, mitoxantrone, natalizumab, ocrelizumab, siponimod, prednisone teriflunomide, opicinumab, and N,N-dimethyl-3β-hydroxycholenamide (DMHCA).

5. Squalene for use according to claim 4, wherein the at least one active agent is selected from interferon beta-1a, interferon beta-1b, peginterferon beta-1a, and N,N-dimethyl-3β-hydroxycholenamide (DMHCA).

6. Squalene for use according to claim 5, wherein a weight ratio of the squalene and the at least one active agent is in a range of 100,000:1 to 10:1.

7. Squalene for use according to any one of claims 1 to 6, wherein 0.1 - 1000 mg/kg squalene is administered per body weight in one day.

8. Pharmaceutical composition comprising squalene or a squalene extract and at least one further active agent selected from the group consisting of alemtuzumab, cladribine, daclizumab, dimethyl fumarate, fingolimod, galtiramer acetate, interferon beta-1a, interferon beta-1b, laquinimod, peginterferon beta-1, mitoxantrone, natalizumab, ocrelizumab, siponimod, prednisone, teriflunomide, opicinumab, and N,N-dimethyl-3β-hydroxycholenamide (DMHCA).

9. Pharmaceutical composition according to claim 8, wherein the at least one active agent is selected from interferon beta-1a, interferon beta-1b, peginterferon beta-1a, and N,N-dimethyl-3β-hydroxycholenamide (DMHCA).

10. Pharmaceutical composition according to claim 8 or 9, wherein the amount of squalene is in a range of 10% to 99% by weight.

11. Pharmaceutical composition according to any one of claims 8 to 10, wherein a weight ratio of the squalene and the at least one active agent is in a range of 100,000:1 to 10:1.

12. Pharmaceutical composition according to any one of claims 8 to 11, wherein the pharmaceutical composition is formulated as capsule, gel, syrup, emulsion or oil.

13. Pharmaceutical composition according to any one of claims 8 to 12, wherein the pharmaceutical composition is administered by oral, dermal, intradermal, intragastral, intracutan, intravasal, intravenous, intramuscular, intraperitoneal, percutan, rectal, subcutaneous, or transdermal application.

14. Pharmaceutical composition according to any one of claims 8 to 13, wherein 0,1 - 1000 mg/kg squalene is administered per body weight in one day.

15. Pharmaceutical composition according to any one of claims 8 to 14 for use in treatment and/or prophylaxis of multiple sclerosis.
